# EUROPEAN PATENT APPLICATION

(11) **EP 0 791 573 A1**
(43) Date of publication of application: **27.08.1997**
(21) Application number: 97101640.7
(22) Date of filing: 03.02.1997
(51) Int. Cl.: C07C 67/62, C07C 69/15

(54) **Nitroxides as antipolymerants for vinyl acetate units**

(30) Priority: 21.02.1996 US 604592
(71) Applicant: Nalco/Exxon Chemicals Company L.P., Sugarland, TX 77478 (US)
(72) Inventor: Clever, Hester A., Sugarland, TX 77478 (US); Lewis, Vincent E., Missouri City, TX 77459 (US); Patel, Natu R., Houston, TX 77043 (US)
(74) Representative: Wibbelmann, Jobst, Dr., Dipl.-Chem.

(57) **Abstract**

The invention comprises a method of inhibiting the polymerization of vinyl acetate in vinyl acetate production units, the method comprising adding from about 0.001 to about 5,000 parts per million of a nitroxide compound to a vinyl acetate production unit whereby the polymerization of vinyl acetate in the unit is inhibited and excess acetaldehyde is not produced.

## Description

### Background of the Invention

### 1. Field of the Invention

The invention relates to a method of inhibiting fouling in vinyl acetate units, and more particularly, to the use of an antipolymerant to prevent fouling in these units.

### 2. Description of the Prior Art

Vinyl acetate plants experience fouling in their product recovery and purification sections due to the free radical polymerization of vinyl acetate. Vinyl acetate producers typically use hydroquinone to control radical polymerization. Acetaldehyde is a contaminant found in the final vinyl acetate product formed by the hydrolysis of vinyl acetate. In this reaction, vinyl acetate reacts with water to give acetic acid and vinyl alcohol. Vinyl alcohol is unstable and tautomerizes to acetaldehyde. Vinyl acetate producers typically have an acetaldehyde specification of 20 - 30 ppm in their final product. If they exceed this amount, their product cannot be sold.

N,N'-Dialkylphenylenediamines (PDA) had been successfully used in this application as polymerization inhibitors. However, they induced excess acetaldehyde formation when used to inhibit vinyl acetate polymerization. PDA appeared to act as a basic catalyst in the hydrolysis of vinyl acetate, as could any amine.

As taught in U.S. Patent No. 4,670,131, issued to Ferrell, stable free radicals, and in particular nitroxide compounds, may be used to inhibit polymerization of olefinic compounds. When 4-hydroxy-2,2,6,6-tetramethylpiperidinyl-1-oxyl, was used to inhibit vinyl acetate polymerization, the hydroxyl group appeared to induce excess acetaldehyde formation. Thus, any other hydroxy substituted nitroxide would also be unsuitable for use as a vinyl acetate polymerization inhibitor. Ideally, any inhibitor used in vinyl acetate production units should not only inhibit vinyl acetate polymerization but should not induce excess acetaldehyde formation.

### Summary of the Invention

The invention comprises a method of inhibiting the polymerization of vinyl acetate in vinyl acetate production units, the method comprising adding from about 0.001 to about 5,000 parts per million of a nitroxide compound to a vinyl acetate production unit whereby the polymerization of reactive monomer in the unit is inhibited and excess acetaldehyde is not produced.

### Description of the Preferred Embodiments

The invention claims the use of various non-hydroxyl-containing, non-amine-containing nitroxides, hereafter referred to as nitroxides, as polymerization inhibitors in concentrations of 0.001 - 5000 parts per million (ppm) in plants producing vinyl acetate. The nitroxides include 2,2,6,6-tetramethylpiperidinyl-1-oxyl, 4-oxo-2,2,6,6-tetramethylpiperidinyl-1-oxyl, esters of 4-hydroxy-2,2,6,6-tetramethylpiperidinyl-1-oxyl where the ester groups may have any substituted moiety attached to the carbonyl group, e.g. OCOCH₃, (acetate), OCO(CH₂)ₙ CH₃ where n = 1 - 20, OCOCH(CH₃)₂ and any other branched hydrocarbon chain, OCOCH₂C₆H₅ and any other substituted aromatic, OCOC₆H₅ and any other substituted aromatic, 3-substituted-2,2,5,5-alkyl-pyrrolidinyl-1-oxyl where the substituent in the 3 position may be H, CN, or any C1 - C20 linear or branched hydrocarbon chain and the alkyl substituents at the 2 and 5 positions may be the same or different C1 - C20 linear or branched hydrocarbon chains, 2,2,4,4-alkyl-oxazolidinyl-3-oxyl where the alkyl groups in the 2 and 4 positions may be the same or different C1 - C20 linear or branched hydrocarbon chain, and di-t-butyl nitroxide. The invention includes all formulations containing the nitroxides in the specified concentration range in various solvents tailored meet the needs of specific units, e.g. water, acetic acid, esters, aromatic solvents, aliphatic hydrocarbon solvents, acetonitrile, vinyl acetate, vinyl chloride and any combination thereof.

In the more preferred embodiment, the nitroxide is added in an amount of from about 1 - 1000 parts per million. In the most preferred embodiment, the nitroxide is added in an amount of from about 25 - 250 parts per million. Preferably, the reactive monomer is added to the product recovery section and the purification section of the vinyl acetate production unit.

The invention also encompasses formulations of the nitroxides with other antipolymerants and/or antioxidants such as hydroquinone, benzoquinone, p-methoxyphenol, 4-t-butylcatechol, 2,6-di-t-butyl-4-methylphenol, phenothiazine, N-alkyl substituted phenylene diamines, tris-nonylphenyl phosphite and related compounds in concentrations ranging from 0.001 - 5000 ppm in the aforementioned solvents in the treatment of the aforementioned vinyl acetate producing plants.

The compositions of the invention advantageously do not form excess acetaldehyde in the vinyl acetate production unit. Moreover, the use of nitroxides in vinyl acetate production units has been counterindicated in the past because of the difficulties inherent in using some nitroxide compositions. Typically, these nitroxides, which contain hydroxyl and amine functional groups react with vinyl acetate to form excess acetaldehyde. The inventors have found that the claimed species are particularly adapted for use in a vinyl acetate production unit because the claimed species do not form the undesirable acetaldehyde.

### Examples

The following examples are presented to describe preferred embodiments and utilities of the invention and are not meant to limit the invention unless otherwise stated in the claims appended hereto. The compounds in this invention have been shown to be polymerization inhibitors, and as the examples below indicate, did not produce acetaldehyde in an amount over and above the untreated sample.

### Example 1

ASTM D-525 was modified to test antifoulants for vinyl acetate. Vinyl acetate (25 mL) containing the desired concentration of antipolymerant was put in a glass liner and inserted into a stainless steel pressure vessel. The apparatus was closed, pressurized to 100 psig with air, and then heated to 100°C. In the modified procedure, the exothermic polymerization caused a large temperature increase. As this was a closed system, a large temperature increase caused a pressure increase inside the vessel. This pressure increase was seen as a spike on the chart recorder trace.

Nitroxide 1 (N1) was evaluated for its effectiveness relative to the parent nitroxide (P1), the parent nitroxide containing either a hydroxyl or an amine functionality. Both of these products were found to be effective at inhibiting vinyl acetate polymerization using the above test. Table 1 gives the results of this evaluation.

**Table 1**

| Compound | Inhibitor Dosage (ppm) | Induction Period (hrs) |
|---|---|---|
| | | |
| Blank | 0 | 3.0 |
| P1 | 50 | 18.0 |
| N1 | 50 | 6.5 |
| N1 | 100 | 9.0 |
| N1 | 200 | >23 |
| | | |

### Example 2

Nitroxide 2 (N2) (50 ppm) was added to vinyl acetate containing benzoyl peroxide initiator (70 ppm). Aliquots (5g) were added to a number of tubes outfitted with Teflon® stopcocks. The samples were degassed three times using the freeze-thaw method and opened under argon. The tubes were placed in an oil bath at 83⁰ C. Tubes were removed at various times and the refractive index of the solution measured. As Table 2 shows, the untreated sample polymerized after 1 hour. The sample treated with Nitroxide 2 polymerized between 7.5 and 8 hours. The nitroxide appeared to be an effective vinyl acetate polymerization inhibitor.

**Table 2**

| Inhibitor | Time (minutes) | Refractive Index |
|---|---|---|
| | | |
| Blank | 0 | 1.3495 |
| Blank | 57 | 1.4274 |
| Blank | 63 | 1.4524 |
| N2 | 0 | 1.3947 |
| N2 | 57 | 1.3495 |
| N2 | 90 | 1.3944 |
| N2 | 154 | 1.3947 |
| N2 | 289 | 1.3948 |
| | | |

### Example 3

Nitroxide 3 (N3) (50 ppm) was added to vinyl acetate containing benzoyl peroxide initiator (70 ppm). Aliquots (5g) were added to 5 tubes outfitted with Teflon stopcocks. The samples were degassed three times using the freeze-thaw method and opened under argon. The tubes were placed in an oil bath at 83⁰ C. Tubes were removed at various times and the refractive index of the solution was measured. The refractive index was plotted as a function of time and compared to results obtained from experiments using Parent Nitroxide 3 (P3) (50 ppm) and a blank using the same procedure. Experiments were done simultaneously. Data is shown in Table 3.

The blank polymerized after approximately one hour. The solution containing Nitroxide 3 (N3) polymerized in 7.5 - 8 hours, indicating that it was effective as a polymerization inhibitor.

**Table 3**

| Nitroxide 3 (50 ppm) | |
|---|---|
| Time (min.) | Refractive Index |
| 0 | 1.3947 |
| 57 | 1.3945 |
| 90 | 1.3944 |
| 154 | 1.3947 |
| 289 | 1.3948 |
| 502 | 1.4201 |

| Parent Nitroxide 3 (50 ppm) | |
|---|---|
| Time (min.) | Refractive Index |
| 0 | 1.3947 |
| 57 | 1.3946 |
| 90 | 1.3943 |
| 154 | 1.3943 |
| 289 | 1.3948 |
| 502 | 1.4001 |

| Blank | |
|---|---|
| Time (min.) | Refractive Index |
| 0 | 1.3945 |
| 57 | 1.4274 |
| 63 | 1.4524 |
| | |

### Example 4

Nitroxide 4 (N4) (40 ppm) was added to vinyl acetate containing benzoyl peroxide initiator (50 ppm). Aliquots (5g) were added to 5 tubes outfitted with Teflon stopcocks. The samples were degassed three times using the freeze-thaw method and opened under argon. The tubes were placed in an oil bath at 83⁰ C. Tubes were removed at various times and the refractive index of the solution was measured. The refractive index was plotted as a function of time and compared to results obtained from experiments using a molar equivalent amount of parent nitroxide (P5) and a blank using the same procedure. Experiments were done simultaneously. Data is shown in Table 4. The nitroxide appeared to be an effective polymerization inhibitor for vinyl acetate.

**Table 4**

| Nitroxide 4 | |
|---|---|
| Time (min.) | Refractive Index |
| 0 | 1.3949 |
| 61 | 1.3943 |
| 139 | 1.3939 |
| 209 | 1.4115 |
| 246 | 1.4130 |
| 265 | 1.4186 |

| Parent Nitroxide 4 | |
|---|---|
| Time (min.) | Refractive Index |
| 0 | 1.3947 |
| 138 | 1.3938 |
| 210 | 1.4107 |
| 248 | 1.4147 |

| Blank | |
|---|---|
| Time (min.) | Refractive Index |
| 0 | 1.3947 |
| 53 | 1.3943 |
| 55 | 1.4292 |
| | |

### Example 5 - Acetaldehyde Production Testing

Solutions of antipolymerants Nitroxide 5 (N5) and Parent Nitroxide 5 (P5) were prepared by adding 0.025g of the specific antipolymerant to 4.975g of vinyl acetate. These solutions were placed in glass tubes fitted with Teflon stopcocks. An untreated sample was also evaluated. The tubes were blanketed with argon, sealed and placed in an 83⁰ C oil bath for 9 days. The tubes were removed from the oil bath, cooled, then analyzed for acetaldehyde content by gas chromatography. Table 5 gives the results.

**Table 5**

| Inhibitor | Time (days) | Acetaldehyde (ppm produced) |
|---|---|---|
| Blank | 9 | 45 |
| P5 | 9 | 171 |
| Blank | 5 | 10 |
| P5 | 5 | 94 |
| Blank | 5 | 10 |
| N5 | 5 | 21 |
| Blank | 5 | 36 |
| N5 | 5 | 45 |
| Blank | 5 | 42 |
| N5 | 5 | 48 |

As the above table indicates, the Parent Nitroxide (P5) induced excess acetaldehyde formation, but the corresponding nitroxide (N5) did not. Thus, these nitroxides were good vinyl acetate polymerization inhibitors that did not induce excess acetaldehyde formation.

The claimed nitroxides of the invention offer the advantage over the parent nitroxides in that they are more soluble in organic solvents. This allows the use of the nitroxide chemistry in systems that require a higher nitroxide concentration than is currently available in hydrocarbon solvents or in systems that do not allow water.

Changes can be made in the composition, operation and arrangement of the method of the present invention described herein without departing from the concept and scope of the invention as defined in the following claims:

## Claims

1. A method of inhibiting the polymerization of vinyl acetate in vinyl acetate production units, the method comprising adding from about 0.001 to about 5,000 parts per million of a compound selected from the group consisting of 2,2,6,6-tetramethylpiperidinyl-1-oxyl, 4-oxo-2,2,6,6-tetramethylpiperidinyl-1-oxyl, esters of 4-hydroxy-2,2,6,6-tetramethylpiperidinyl-1-oxyl where the ester groups may have any substituted moiety attached to the carbonyl group, e.g. OCOCH₃, (acetate), OCO(CH₂)ₙ CH₃ where n = 1 - 20, OCOCH(CH₃)₂ and any other branched hydrocarbon chain, OCOCH₂C₆H₅ and any other substituted aromatic, OCOC₆H₅ and any other substituted aromatic, 3-substituted-2,2,5,5-alkyl-pyrrolidinyl-1-oxyl where the substituent in the 3 position may be H, CN, or any C1 - C20 linear or branched hydrocarbon chain and the alkyl substituents at the 2 and 5 positions may be the same or different C1 - C20 linear or branched hydrocarbon chains, 2,2,4,4-alkyl-oxazolidinyl-3-oxyl where the alkyl groups in the 2 and 4 positions may be the same or different C1 - C20 linear or branched hydrocarbon chain, and di-t-butyl nitroxide to a vinyl acetate production unit whereby the polymerization of reactive monomer in the unit is inhibited and whereby an excess of acetaldehyde is not formed within the vinyl acetate production unit.

2. The method of Claim 1, wherein the compound is added in a section of the vinyl acetate production unit selected from the group consisting of the purification section and the product recovery section.

3. The method of Claim 1 wherein the reactive monomer is dissolved in a solvent selected from the group consisting of water, acetic acid, esters, aromatic solvents, aliphatic hydrocarbon solvents, acetonitrile, vinyl acetate and any combination thereof.

4. The method of Claim 1 further comprising the addition of an antipolymerant selected from the group consisting of hydroquinone, benzoquinone, p-methoxyphenol, 4-t-butylcatechol, 2,6-di-t-butyl-4-methylphenol, phenothiazine, N-alkyl substituted phenylene diamines, tris-nonylphenyl phosphite and related compounds in concentrations ranging from 0.001 - 5000 parts per million.
